# EUROPEAN PATENT APPLICATION

(11) **EP 3 459 559 A1**
(43) Date of publication of application: **27.03.2019**
(21) Application number: 17192428.5
(22) Date of filing: 21.09.2017
(51) Int. Cl.: A61K 38/22, A61K 51/08, C07K 14/595, G01N 33/574

(54) **MINIGASTRIN DERIVATES, IN PARTICULAR FOR USE IN CCK2 RECEPTOR POSITIVE TUMOUR DIAGNOSIS AND/OR TREATMENT**

(71) Applicant: PAUL SCHERRER INSTITUT, 5232 Villigen PSI (CH)
(72) Inventor: BEHE, Martin, 4460 Gelterkinden (CH)
(74) Representative: Fischer, Michael

(57) **Abstract**

It is therefore the objective of the present invention to provide minigastrin derivates which further improve the accumulation in CCK-2 receptor positive tumours by simultaneously very low accumulation in other organs, e.g. the kidneys.

This objective is achieved according to the present invention by a minigastrin derviate having the formula: X-Z-Ala-Tyr-Gly-Trp-Met-Asp-Phe-NH₂ (Y),
wherein at least one of the connecting or terminal amide bonds between, before or after the amino acids of the sequence Z, Ala, Tyr, Gly, Trp, Met, Asp, Phe and NH₂ or Y (C-terminal) is replaced by a 1,4-disubstituted or a 1,5-disubstituted 1,2,3-triazole, while X stands for a chemical group attached to the peptide for the purpose of diagnostic and/or therapeutic intervention at CCK-2 receptor relevant diseases, Y stands for C-terminal modifications of the peptide, such as amide, primary and secondary amides, free carboxylic acids and carboxylic ester derivatives including but not limited to amides and esters derived from linear or branched alkyl-,alkenyl-, alkynyl- aromatic-, and heterocyclic alcohols, and Z stands for a linker or DGlu* wherein DGlu* stands for a chain of DGlu having 1 to 6 repetitions (-DGlu- to -DGlu-DGlu-DGlu-DGlu-DGlu-DGlu-).

These minigastrin derivates have a high specific internalization, excellent IC₅₀ values and sufficient plasma stability.

## Description

The present invention relates to minigastrin derivates and their use in CCK2 receptor positive tumour diagnosis and/or treatment.

G protein-coupled receptors (GPCR) are used as target proteins for radiolabelled peptides since the early 90's. The somatostatin receptor was the prototype for radionuclide imaging and therapy with peptides (Lit) resulting in a clinical first line therapies for neuroendocrine tumors with Y-90 and Lu-177 labelled derivatives of octreotide (Lit). Several radiolabelled peptides were tested for the possibility to target overexpressed GPCR on tumours including gastrin realising peptide analogues (GRP), glucagon-like peptide 1 analogues (GLP-1), neurotensin analogues (NT) or neuropeptide Y analogues (NPY) (Mäcke, Reubi J Nucl Med 2008; 49:1735-1738). An additional very interesting target is the cholecystokinin-2 receptor (CCK-2 R). This receptor is mainly expressed on medullary thyroid carcinomas (MTC), small cell lung cancers (SCLC) and stromal ovarial tumors (Reubi, Int J Cancer. 1996 and Reubi, Cancer Res. 1997). Radiolabelled gastrin analogues are good candidates for targeting imaging and therapy. It was shown that In-111 labelled gastrin analogues are superior for detecting MTC compared to OctreoScan-111 and give additional information on neuroendocrine tumours particularly if they are negative in somatostatin receptor scintigraphy (Endocr Relat Cancer. 2006 Dec;13(4) :1203-11.; Eur J Nucl Med Mol Imaging. 2006 Nov;33(11):1273-9).

But due to the high kidney uptake the radiolabelled peptides could not be used for therapy. The high kidney uptake is correlated with the six negatively charged glutamic acids. 12 gastrin related compounds were designed, synthesised and compared as 111In labelled compounds. The best compounds with respect to a high tumour to kidney ratio are the minigastrins with six D-glutamic acids or six glutamines. These compounds still possess a methionine which can be oxidised easily. This is a disadvantage for clinical application because the receptor affinity is dramatically decreased after oxidation of the methionine and the production under GMP may be hampered dramatically.

A high potential for a significant improvement of the therapy and the image generation with patients having metastasized medullary thyroid carcinomas (MTC), small cell lung cancers (SCLC) and further CCK-2 receptor positive tumours has a specific labelling of the tumour cells with radio-labelled gastrin analogue. Basis for this finding is the proof of an over-expression of the respective CCK-2 target receptor at 92% of the investigated MTC, said proof being yielded by in-vitro studies [Reubi 1997]. Furthermore, the same working group identified the same over-expression of the CCK-2 target receptor at 57% of small cell lung cancers, 65% of astrocytomes and 100% of stromal ovarial tumours.

First therapy studies (phase 0 study) had been executed at eight patients having advanced metastasized medullary thyroid carcinomas. For two patients a partial remission was achieved, four patients showed a stabilization of the formerly strongly progressive course of the cancer disease MTC after a therapy with ⁹⁰Y-labelled minigastrin analogue. This study had to be stopped due to the nephrotoxicity of the therapy in terms of a strong accumulation of the substances used in said assay in the kidneys.

With support of the European COST initiative (European Cooperation in Science and Technology), in the meantime a plurality of significantly improved radio-labelled gastrin-analogues have been synthesized by various working groups and have been investigated for their characteristics. As compared to the old gastrin analogue, these younger substances possess a significantly higher tumour-to-kidney ratio with respect to the absorption in human tissue [Laverman 2011, Polenc-Peitl 2011, Ocak 2011, Fani 2012]. Currently, out of these younger gastrin analogues, ¹⁷⁷Lu-PP-F11 (the linear minigastrin analogue with six D-Glu residues, hereinafter called PP-F11) exhibited best properties for future radio nuclide therapy due to its high favorable accumulation in the tumor accompanied by a low accumulation in the kidneys.

This PP-F11 minigastrin analogue has been further improved according to the international patent application WO 2015/067473 A1 wherein the minigastrin analogue PP-F11 having the formula: X-DGlu-DGlu-DGlu-DGlu-DGlu-DGlu-Ala-Tyr-Gly-Trp-Y-Asp-Phe-NH₂, wherein Y stands for an amino acid replacing methionine and X stands for a chemical group attached to the peptide for the purpose of diagnostic and/or therapeutic intervention at CCK-2 receptor relevant diseases. In particular, very suitable compounds with respect to a high tumour to kidney ratio are minigastrin derivates with six D-glutamic acids or six glutamines. These compounds still possess a methionine which can be oxidised easily which is a disadvantage for clinical application under GMP due to the forms which may occur. Therefore, the replacement of the methionine by a non oxidizable isosteric amino acids but retaining the biological activity leads to a compound with no oxidation potential. This avoids the oxidation during storage and production which could be lead to lower affinity compound resulting in a low tumor to kidney ratio. In a preferred embodiment according to WO 2015/067473 A1, the methionine is replaced by norleucine. This so-called PP-F11N minigastrin exhibits excellent tumour-kidney-ratio and is therefore one very promising candidate for clinical applications.

It is therefore the objective of the present invention to provide minigastrin derivates which further improve the accumulation by the metabolic stabilization of the peptide or improving its receptor affinity and specificity in CCK-2 receptor positive tumours by simultaneously very low accumulation in other organs, e.g. the kidneys.

This objective is achieved according to the present invention by a minigastrin derviate having the formula: X-Z-Ala-Tyr-Gly-Trp-Met-Asp-Phe-NH₂ (Y), wherein at least one of the connecting or terminal amide bonds between, before or after the amino acids of the sequence Z, Ala, Tyr, Gly, Trp, Met, Asp, Phe and NH₂ or Y (C-terminal) is replaced by a 1,4-disubstituted or a 1,5-disubstituted 1,2,3-triazole, while X stands for a chemical group attached to the peptide for the purpose of diagnostic and/or therapeutic intervention at CCK-2 receptor relevant diseases, Y stands for C-terminal modifications of the peptide, such as amide, primary and secondary amides, free carboxylic acids and carboxylic ester derivatives including but not limited to amides and esters derived from linear or branched alkyl-,alkenyl-, alkynyl- aromatic-, and heterocyclic alcohols, and Z stands for a linker or DGlu* wherein DGlu* stands for a chain of DGlu having 1 to 6 repetitions (-DGlu- to -DGlu-DGlu-DGlu-DGlu-DGlu-DGlu-).

These minigastrin derivates have a high receptor specific cell internalization, excellent IC₅₀ values towards CCK2 and sufficient plasma stability.

Preferably, methionine is replaced by norleucine or another amino acid which conserve the affinity to the receptor, preferably to give minigastrin derivate [Nle¹⁵]-MG11 having one DGlu only.

With respect to radio cancer treatments, X may stand for a radionuclide including the attachment group like a chelator for radiometals such as ¹⁷⁷Lu or ⁹⁰Y or ¹¹¹In, or a prostethic group for non-metals like ¹⁸F , 11C, or radioiodines or a functional group suitable for pre-targeting approaches. In order to improve the medical imaging, the X may stand for an optically active chemical compound, such Alexa Fluor® 647, IRDye 680RD, DY-700 or any other photoactive substance and for optical therapeutic application it may be a photosensitizer like Photofrin, Forscam or Photochlor. For both applications the active chemical compound may be an optical active nanopartical. In order to support the chemotherapeutic intervention, X may stand for a chemotherapeutic active compound, such as gemcitabine, doxorubicine or cyclophosphamide. X may also stand for a combination of an imaging agent (dye, radionuclide) and a therapeutic entity (cytotoxic compounds, radionuclide). The delivery of the described agents may be done by a nanoparticle or liposome as X whereas they are loaded with chemotherapeutic agents.

Z may also present a linker or spacer unit that covalently connects the peptide with the imaging probe or therapeutic while keeping them at distance in order to avoid potential interference with the biological properties of the peptide.

Linker moieties may include but are not limited to linear or branched alkyl chains of 1-20 carbon length containing but not limited to 1-10 heteroatoms (including O, S, N, P), all carbon or heterocyclic aromatic moieties such as phenyl, naphthalene, triazoles, thiophenes, furans etc., and unsaturated C-C or C-heteroatom (O, N, S, P) bonds either in the main linker chain or in one or several sidechains.

Preferably, X stands for a chelator wherein one chelator for radiometals can be DOTA (1,4,7,10-tetraazacyclododecane-1,4,7,10-tetraacetic acid) and/or the radionuclide is selected from the group consisting of ¹⁷⁷Lu, Ga-68/67, ⁹⁰Y and ¹¹¹In. Other radionuclides (Tc-99m, Cu-64/67, Ac-225, Bi-213, Pb-212, Th-227) and other suitable chelators are known to those skilled in the art, such as MAG3, HYNIC, NOTA, NODAGA, DOTAGA, CHX-A"-DTPA, DFO, TCMC, HEHA, sarcophagines, cross-bridged versions of cyclam and cylcen chelators.

Suitable minigastrin derivates are PP-F11N and [Nle¹⁵]-MG11 which are defined as:
DOTA-DGlu-DGlu-DGlu-DGlu-DGlu-DGlu-Ala-Tyr-Gly-Trp-Nle-Asp-Phe-NH₂ (PP-F11N) and
DOTA-DGlu-Ala-Tyr-Gly-Trp-Nle-Asp-Phe-NH₂ ([Nle¹⁵]-MG11).

Preferably, X stands for a nanoparticle or a liposome which have a diagnostic function (e.g. optical active or MRI contrast agent) or which have therapeutic function by themselves or are loaded with an active compound.

With respect to the use of the inventive minigastrin derivate, a diagnostic intervention at CCK-2 receptor relevant diseases and/or a therapeutic intervention at CCK-2 receptor relevant diseases is/are foreseen.

Preferred embodiments of the present invention are hereinafter described in more detail with respect to the attached drawings which depict in:
- Figure 1: the specific internalisation of [Nle¹⁵]-MG11 in its derivated forms according to the termed triazole-scan;
- Figure 2: the half maximal inhibitory concentration IC₅₀ of [Nle¹⁵]-MG11 in its derivated forms according to the termed triazole-scan;
- Figure 3: the plasma stability of [Nle¹⁵]-MG11 in its derivated forms according to the termed triazole-scan;
- Figure 4: the summary of the results shown in Figures 1 to 3;

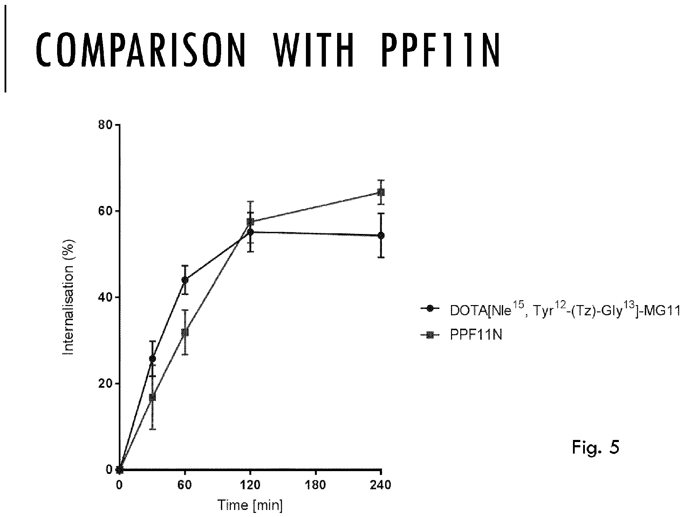

- Figure 5: a comparison of PP-F11N and the [Nle¹⁵]-MG11 derivate DOTA [Nle¹⁵, Tyr¹²- (Tz) -Gly¹³] -MG11; and
- Figure 6: principally the triazole scan with DOTA[Nle¹⁵]-MG11.

[Nle¹⁵]-MG11 is a truncated analogue of minigastrin, a regulatory peptide with high affinity and specificity towards the cholecystokinin 2 receptor (CCK2R) which is overexpressed in various types of cancer. The N-terminal conjugation of 1,4,7,10-tetraazacyclododecane-1,4,7,10-tetraacetic acid (DOTA) allows for radiolabelling of the peptide with metallic radionuclides (e.g., ¹⁷⁷Lu) and subsequent use for *in vivo* tumour imaging and peptide receptor radionuclide therapy.

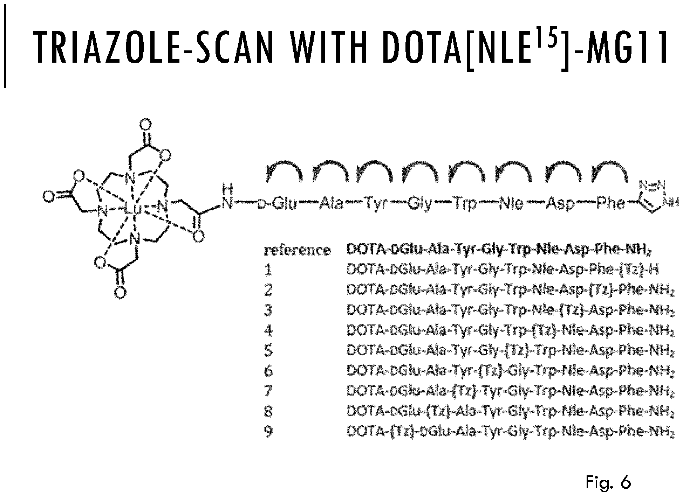

A drawback of using MG11 as a tumour-targeting vector is its low tumour uptake due to fast enzymatic degradation which results in a biological half-life of only a few minutes. The systematic replacement of single amide bonds in the peptide sequence by stable 1,4-disubstituted or 1,5-disubstituted 1,2,3-triazoles, termed a triazole-scan, leads to an improved proteolytic stability and tumour targeting properties. To prove the general utility of this new methodology, a triazole-scan was performed with DOTA[Nle¹⁵]-MG11 by a solid phase approach employing a combination of SPPS and the Copper(I)-catalysed azide-alkyne cycloaddition (CuAAC for 1,4-disubstituted 1,2,3-triazoles) as this systematically shown in Figure 6. The introduction of 1,5-disubstituted 1,2,3-triazoles is analogous but employing the Ruthenium-catalysed azide-alkyne cycloaddition (RuAAC).

The following examples discuss the synthesis of radiolabelled triazolopeptidomimetics and the evaluation of their physiochemical properties. Receptor affinities (IC₅₀), tumour cell internalisation rates, and plasma stabilities of the peptide conjugates were investigated *in vitro.* First results of *in vivo* studies with xenografted mice have been achieved. The ultimate goal of this project is to identify an analogue of DOTA[Nle¹⁵]-MG11 or PPF11N with maintained or improved biological activity or superior resistance to enzymatic degradation leading to improved uptake in tumours.

The building blocks for CuAAC and triazolopeptides have been prepared by adapted procedures reported in literature by Valdere et al. and Mascarin et al.

### General Procedures A: Synthesis of Weinreb Amides

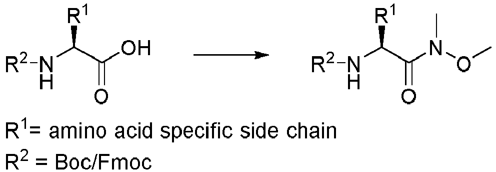

The corresponding Fmoc- or Boc- protected amino acid (1 equiv.) was dissolved in CH₂Cl₂ (0.1 M) and DIPEA (2.5 equiv.) and BOP (1 equiv.) were added. The solution was stirred for 15 min, N,O-dimethylhydroxylamine (1.2 equiv.) was added and the reaction was stirred for 12-14 h at rt. The solution was diluted with CH₂Cl₂, washed with 0.1 M HCl (3x), saturated aq. NaHCO₃ (3x) and water (3x). The organic phase was dried over MgSO₄, filtered, and the solvent was removed under reduced pressure. The desired Weinreb amide was isolated by flash chromatography on silica gel.

### General Procedures B: Synthesis of α-Amino Alcohols

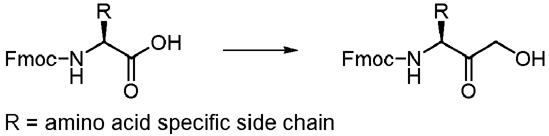

For amino acids with a protected carboxylic acid in their side chain, the corresponding Fmoc-protected amino acid (1 equiv.) was dissolved in anhydrous THF (0.2 M) under Argon and cooled to 0 °C (ice bath). N-Methylmorpholine (1.1 equiv.) and isobutyl chloroformate (1.05 equiv.) were added and the reaction was stirred for 15 min at 0 °C. Completion of the reaction was monitored by TLC. The white suspension was added dropwise to a precooled suspension of NaBH₄ (2 equiv.) in THF/MeOH (3:1, or pure THF) at -78 °C (dry ice, acetone) and stirred for 20 min. Upon completion of the reduction, residual hydrides were quenched by aq. 10% acetic acid and the solution was concentrated under reduced pressure. The residue was extracted with ethylacetate (3x), and the organic layer was washed with aq. sat. NaHCO₃ (2x) and water (1x). The organic layer was dried over Na₂SO₄, filtered and the solvent was removed under reduced pressure. The desired α-amino alcohol was isolated by flash chromatography on silica gel.

### General Procedures C: Synthesis of α-Amino Alkynes5

### C.1 Synthesis of α-Amino Alkynes from Boc-protected Weinreb amides

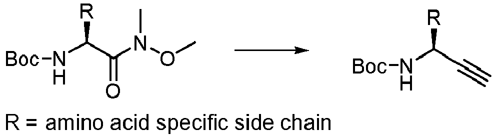

The corresponding Weinreb amide (1 equiv.) was placed in a flame dried flask under argon and dissolved in anhydrous CH₂Cl₂ (0.1 M). The solution was cooled to -78 °C (dry ice/acetone bath) and 1 M DIBAL-H in toluene was added dropwise (3 equiv.). After 1 h of stirring, the reaction was checked for completion by TLC. If the reaction was not finished, 1 M DIBAL-H in toluene (1 equiv.) was added and the reaction was stirred again for 1 h at -78 °C. After consumption of the starting material, the excess hydride was quenched by slow addition of anhydrous MeOH and the reaction was allowed to warm to 0 °C (ice/water bath). K₂CO₃ (3 equiv.), dimethyl-(1-diazo-2-oxopropyl)phosphonate (2 equiv.) and anhydrous MeOH were added and the reaction mixture was stirred for 12-14 h at rt. A saturated solution of Rochelle's salt was added and the mixture was stirred for 30 min. The solution was diluted with water and CH₂Cl₂ and the aqueous phase was extracted with CH₂Cl₂ (3x). The combined organic phases were dried over Na₂SO₄ , filtered, and the solvent was removed under reduced pressure. The desired alkyne was isolated by flash chromatography on silica gel.

### C.2 Synthesis of α-Amino Alkynes from Fmoc-protected Weinreb amides

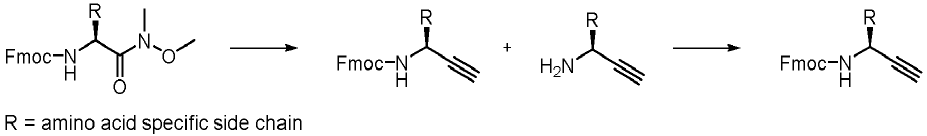

The corresponding Weinreb amide (1 equiv.) was placed in a flame dried flask under argon and dissolved in anhydrous CH₂Cl₂ (0.1 M). The solution was cooled to -78 °C (dry ice/acetone bath) and 1 M DIBAL-H in toluene was added dropwise (3 equiv.). After 1 h of stirring, the reaction was checked for completion by TLC. If the reaction was not finished, 1 M DIBAL-H in toluene (1 equiv.) was added and the reaction was stirred again for 1H at -78 °C. After consumption of the starting material, the excess hydride was quenched by slow addition of anhydrous MeOH and the reaction was allowed to warm to 0 °C (ice/water bath). K₂CO₃ (3 equiv.), dimethyl-(1-diazo-2-oxopropyl)phosphonate (2 equiv.) and anhydrous MeOH were added and the reaction mixture was stirred for 12-14 h at RT. A saturated solution of Rochelle's salt was added and the mixture was stirred for 30 min. The solution was diluted with water and CH₂Cl₂ and the aqueous phase was extracted with CH₂Cl₂ (3x). The combined organic phases were dried over Na₂SO₄, filtered, and the solvent was removed under reduced pressure. If cleavage of the Fmoc protecting group was detected by TLC, the crude mixture was dissolved in CH₂Cl₂ (0.1 M according to initial scale). DIPEA (2.5 equiv.) and Fmoc-OSu (2 equiv.) were added and the reaction was stirred for 12-14 h at rt. The reaction mixture was then diluted with CH₂Cl₂ and water. The aqueous phase was extracted with CH₂Cl₂ three times. The combined organic phases were dried over Na₂SO₄, filtered, and the solvent was removed under reduced pressure. The desired alkyne was isolated by flash chromatography on silica gel.

### C.3 Synthesis of α-Amino Alkynes from Fmoc-protected α-Amino Alcohols

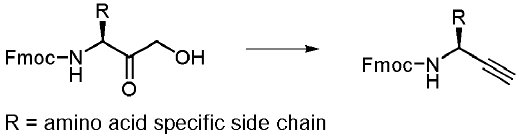

DMSO (2.2 equiv.) was dissolved in anhydrous CH₂Cl₂ (1 M) and cooled to -45 °C (dry ice/MeCN bath) under Argon. Oxalyl dichloride (1.2 equiv.) was added dropwise at -45 °C under development of gas. The solution was stirred for 5 min, before the corresponding Fmoc-protected α-amino alcohol (1 equiv., 0.13 M in CH₂Cl₂) was added dropwise at -45 °C and stirred for 30 min. DIPEA (3 equiv.) was added, the reaction was warmed to -20 °C (NaCl/ice bath) and monitored by TLC until completion. The solution was then diluted with CH₂Cl₂ and the organic layer was extracted with water, 1 M NaHSO₄ and water. The combined organic phases were dried over Na₂SO₄, filtered, and the solvent was removed under reduced pressure. The crude reaction workup was subsequently dissolved in anhydrous MeOH (0.1 M according to initial yield), K₂CO₃ (3 equiv.) and dimethyl-(1-diazo-2-oxopropyl)phosphonate (2 equiv.) were added and the reaction mixture was stirred for 12-14 h at RT. The reaction mixture was diluted with water and the aqueous phase was extracted with CH₂Cl₂ three times. The combined organic phases were dried over Na₂SO₄, filtered, and the solvent was removed under reduced pressure. If cleavage of the Fmoc protecting group was detected by TLC, the crude mixture was dissolved in CH₂Cl₂ (0.1 M according to initial scale). DIPEA (2.5 equiv.) and Fmoc-OSu (2 equiv.) were added and the reaction was stirred for 12-14 h at rt. The reaction mixture was then diluted with CH₂Cl₂ and water. The aqueous phase was extracted with CH₂Cl₂ three times. The combined organic phases were dried over Na₂SO₄, filtered, and the solvent was removed under reduced pressure. The desired alkyne was isolated by flash chromatography on silica gel.

### General Procedure D for the Determination of the Optical Purity of α-Amino Alkyne Building Blocks: Synthesis of Dipeptides from α-Amino Alkynes

### D.1 Synthesis of Dipeptides from Boc-protected α-Amino Alkynes

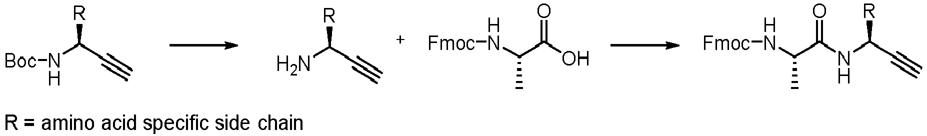

The corresponding α-amino alkyne (1 equiv.) was dissolved in a solution of CH₂Cl_{2/}TFA/H₂O (75:20:5) (0.05M) and the reaction was stirred for 15-30 min. after completion of the reaction, the solvent was removed under reduced pressure. Residual amounts of water and TFA were removed by coevaporation with toluene. The residue was dissolved in CH₂Cl₂ (0.1 M) and PG-Ala-OH (2 equiv., PG = Boc or Fmoc), (benzotriazol-1-yloxy)tris(dimethylamino)phosphonium hexafluorophosphate (BOP, 2 equiv.) and DIPEA (5 equiv.) were added. The reaction was stirred at rt and monitored by TLC until completion. The solvent was removed from the crude mixture under reduced pressure and the desired dipeptide was isolated by flash chromatography on silica gel.

### D.2 Synthesis of Dipeptides from Fmoc-protected α-Amino Alkynes

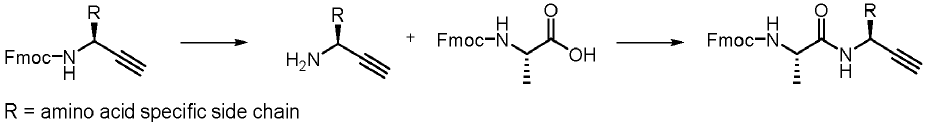

The corresponding α-amino alkyne (1 equiv.) was dissolved in 25% piperidine in DMF (0.05 M) and the reaction was stirred for 15-30 min at rt. Ice-cold H₂O was added to the reaction mixture and it was extracted with EtOAc (3x). The combined organic fractions were dried over MgSO₄, filtered, and the solvent was removed under reduced pressure. The residue was dissolved in CH₂Cl₂ (0.1 M) and Fmoc-Ala-OH (2 equiv.), BOP (2 equiv.) and DIPEA (5 equiv.) were added. The reaction was stirred at rt and monitored by TLC until completion. The solvent was removed from the crude mixture under reduced pressure and the desired dipeptide was isolated by flash chromatography on silica gel.

General Procedures E: Manual Solid Phase Peptide Synthesis The rink amide MBHA LL resin (ca. 100 mg, 0.03 - 0.04 mmol) was placed in a polypropylene syringe with a polyethylene frit and a Teflon tap and swollen repeatedly in CH₂Cl₂ and DMF. 20% piperidine in DMF was used to cleave the Fmoc protecting group (3 x 3 min, rt). For elongation of the sequence, the Fmoc-protected amino acids or DOTA-tris(tert-butyl ester) (2 equiv., 0.06 mmol), HATU (1.9 equiv., 0.057 mmol) and DIPEA (5 equiv., 0.15 mmol) in DMF (total 3 mL) were added to the resin. The suspension was shaken for 1 h at rt. The solvent was removed by filtration, and the resin was repeatedly washed with DMF and CH₂Cl₂. Completion of the reaction was monitored by the Kaiser test and the coupling was repeated if necessary.

### General Procedure F: Introduction of the Azido Functionality on the N-Terminus of the Peptide on Solid Support

After cleavage of the Fmoc protecting group, the free N-terminal amine was treated with imidazole-1-sulfonyl azide hydrochloride (3 equiv., 0.09 mmol), DIPEA (9 equiv., 0.27 mmol) and a catalytic amount of CuSO₄ (0.01 equiv., 0.03 µmol)7 in DMF (total 2 mL). The suspension was shaken for 1 h at rt. The solvent was removed by filtration, and the resin was repeatedly washed with a 0.5% solution of sodium diethyldithiocarbamate in DMF, DMF and CH₂Cl₂. Completion of the reaction was monitored by the Kaiser test and repeated if necessary.

### General Procedure G: Solid Phase Copper(I)-Catalysed Cycloaddition (CuAAC)

The Fmoc-protected α-amino alkyne (2 equiv., 0.06 mmol), DIPEA (1 equiv., 0.03 mmol), tetrakis(acetonitrile)copper(I) hexafluorophosphate (0.5 equiv., 0.015 mmol) and tris[(1-benzyl-1H-1,2,3-triazol-4-yl)methyl]amine (TBTA, 0.5 equiv., 0.015 mmol) in DMF (2 mL) were added to the N-terminal azide functionality and the suspension was shaken for 12-14 h at rt. The solvent was removed by filtration, and the resin was repeatedly washed with a 0.5% solution of sodium diethyldithiocarbamate in DMF, DMF and CH₂Cl₂. Completion of the reaction was monitored by a colorimetric test for aliphatic azides.

### General Procedure H: Solid Phase Ruthenium-Catalysed Cycloaddition (RuAAC)

The Fmoc-protected α-amino alkyne (2 equiv., 0.06 mmol) and (chloro(pentamethylcyclopentadienyl) (cyclooctadiene)rutheniu m (CpRu(COD)Cl, 0.5 equiv.)in DMF (2 mL) were added to the N-terminal azide functionality under argon atmosphere and the suspension was shaken for 12-14 h at rt. The solvent was removed by filtration, and the resin was repeatedly washed with a 0.5% solution of sodium diethyldithiocarbamate in DMF, DMF and CH₂Cl₂. Completion of the reaction was monitored by a colorimetric test for aliphatic azides

### General Procedure I: Cleavage and Purification of the Peptide Conjugates

After the final coupling of the N-terminal chelator DOTA-tris(tert-butyl ester), the conjugates were deprotected and cleaved from the resin using TFA/TIS/H₂O/phenol (92.5/2.5/2.5/2.5, 6 mL) with agitation for 5 h at rt. The cleavage mixture was separated from the resin by filtration and a stream of nitrogen was applied for evaporation of the volatile components. The crude peptide was then precipitated by the addition of ice-cold diethyl ether (15 mL). After centrifugation (1800 rpm, 5 min) and two washing steps with ice-cold diethyl ether, the crude peptide conjugates were dissolved in 20% CH₃CN in water (1 mg/mL) and purified by reverse phase semipreparative HPLC. Subsequent lyophilisation gave the final products as white powders.

### Synthesis Results are shown in Table 1.

**Table 1: Summary of synthesis scale, yields and purities of the novel triazolominigastrins**

| | Mwt (g/mol) | scale (mmol) | yield (%) | purity (%) |
|---|---|---|---|---|
| DOTA[Nle15]-MG11 | 1384.6 | 0.058 | 67.4 | > 99 |
| DOTA[Nle15, Phe17-(Tz)-H]-MG11 | 1409.5 | 0.0511 | 35.7 | > 99 |
| DOTA[Nle15, Asp16-(Tz)-Phe17]-MG11 | 1409.5 | 0.0379 | 10.1 | 98.5 |
| DOTA[Nle15, Nle15-(Tz)-Asp16]-MG11 | 1409.5 | 0.0399 | 76.3 | > 99 |
| DOTA[Nle15, Trp14-(Tz)-Nle15]-MG11 | 1409.5 | 0.041 | 61.8 | 97 |
| DOTA[Nle15, Gly13-(Tz)-Trp14]-MG11 | 1409.5 | 0.0324 | 12.5 | 98.6 |
| DOTA[Nle15, Tyr12-(Tz)-Gly13]-MG11 | 1409.5 | 0.04101 | 13.9 | > 99 |
| DOTA[Nle15, Ala11-(Tz)-Tyr12]-MG11 | 1409.5 | 0.0394 | 23.7 | > 99 |
| DOTA[Nle15, DGlu10-(Tz)-Ala11]-MG11 | 1409.5 | 0.0403 | 12.5 | > 99 |

### (Radio)metal Labelling

Stock solutions were prepared by dissolving the reference substances (DOTA-[Nle15]-MG11 and DOTA-PP-F11-N) or the triazolominigastrins (1 mg, 750 nmol) in ammonium acetate buffer (50 µL, 0.5 M, pH 5.5) and addition of water to a final peptide concentration of 250 µM (approx. 0.3 mg/mL). For in vitro experiments, DOTA-functionalized compounds (1 nmol, 4 µL of 250 µM stock solution) were added to a mixture of aq. HCl (22.5 µL, 0.05 M, pH 1.3), ammonium acetate buffer (10 µL, 0.5 M, pH 5.5) and aq. sodium ascorbate (5 µL, 0.5 M). 20-25 MBq of 177LuCl3 (ca. 2.5 µL, in 0.04 M HCl, 20-25 MBq/nmol) were added and the mixtures were heated to 95 °C for 20 min in a heating block. After labelling, a 1 µL aliquot of the labelling mixture was added to aq. DTPA (200 µL, 25 µM) for quality control by γ-HPLC.

For the labelling with non-radioactive 175Lu, the test compounds (25 nmol, 100 µL, 250 µM) were mixed with a 5-molar excess of aq. ¹⁷⁵LuCl₃ (125 nmol, 12.5 µL, 10 mM), ammonium acetate (5 µL, 0.5 M, pH 5.5) and heated to 95 °C for 20 min in a heating block.

For in vivo experiments, DOTA-functionalized compounds were labelled with higher amount of ¹⁷⁷LuCl₃ resulting in specific activities of 45-55 MBq/nmol and - after quality control - diluted with PBS to reach concentrations of 100 pmol/mL.

### Cell Culture

Human Medullary Thyroid Cancer cells (MZ-CRC1) expressing the CCK2R were grown in monolayers in Nunclon™ Delta treated cell culture flasks in humidified air at 5% CO₂ and 37 °C. The cells were maintained in the culture medium DMEM (high glucose (4.5 g/L)) supplemented with 20 mM L-Glutamine (L-Glu) and 10% FCS. The culture was passaged regularly at 80 to 90% confluency using a 0.25% trypsin 0.38‰ EDTA solution. Assays were conducted in the assay medium DMEM (high glucose) containing 0.1% BSA.

### Cell Internalization Experiments

On the day prior to the experiment MZ-CRC1 cells were placed in six-well plates (0.85·106 cells/well) in cell culture medium and incubated overnight for attachment. On the day of the experiment, the medium was removed and the cells were washed twice with 1 mL PBS. The plates were put on ice for preparation. 0.9 mL of assay medium was dispensed to all wells except the ones for nonspecific binding. 0.2 pmol of 177Lu-labelled conjugates (100 µL, 2 nM in assay medium, ca. 4.2 kBq) were dispensed to all wells. For the determination of nonspecific binding, a 5000-fold excess of minigastrin (1 nmol, 100 µL, 10 µM in assay medium) was added to 0.8 mL of assay medium containing the ¹⁷⁷Lu labelled conjugates. The plates were incubated at 37 °C in 5% CO₂ to allow binding and internalization. The process was stopped after 30, 60, 120 and 240 min by collection of the supernatant. The cells were washed twice with PBS (each 0.6 mL). The combined supernatants represent the free, unbound fraction of radioactivity. Membrane-bound activity was determined by incubating the cells with cold saline glycine buffer (0.6 mL, 0.05 M, pH 2.8) twice for 5 min at rt. The internalized fraction was isolated by two cycles of cell lysis with NaOH (each 0.6 mL, 1 M, 10 min, rt). The radioactivity of the fractions was measured by a COBRA-II gamma counter and is represented as percentage of total applied radioactivity dosage (n=3-5 in triplicates).

### Receptor Affinity - IC50 Assays

On the day prior to the experiment MZ-CRC1 cells were placed in six-well plates (0.85·106 cells/well) in cell culture medium and incubated overnight for attachment. On the day of the experiment, the medium was removed and the cells were washed twice with 1 mL PBS. On ice, 0.8 mL of assay medium and the radiolabelled reference compound ¹⁷⁷Lu-DOTA-PP-F11N (0.2 pmol, 2 nM in assay medium, 100 µL, ca. 4.2 kBq) were dispensed to each well (final concentration in well = 0.2 nM). ¹⁷⁵Lu-labelled test compounds were added to reach final well concentrations of 10⁻¹¹ to 5·10⁻⁶ M (100 µL of dilution series from 10⁻¹⁰ to 5·10⁻⁵ M in assay medium). Total binding of 177Lu-DOTA-PP-F11N was identified by incubation of the cells without addition of test compounds. After incubation of the plates at 4 °C for 1 h, the supernatant was removed and cells were washed twice with 1 mL cold PBS. NaOH was added twice to all wells for cell lysis (0.6 mL, 1 M, 10 min, rt). The radioactivity associated with the lysed cells was determined by a COBRA-II gamma counter. 50% inhibitory concentrations (IC50) were calculated by normalized nonlinear regression with GraphPad Prism (n=3 in triplicates).

### Blood Plasma Stability

The ¹⁷⁷Lu-labelled compounds were diluted with 0.9% NaCl to a concentration of 3.75 µM and incubated (375 pmol, 100 µL, 7.5-12 MBq) in nitrogen-flushed fresh human blood plasma (1.5 mL) at 37 °C. At different time points (0.5, 1, 2, 4, 6 and 24 h) aliquots (75 µL) were taken and the proteins were precipitated in CH₃CN (100 µL) and centrifuged (2 min, 14680 rpm, rt). The supernatant (75 µL) was diluted with water (75 µL) and analyzed by γ-HPLC. One phase decay nonlinear regression (A = A0 * e^{-kT}) was used to calculate the half-lifes (t_{1/2}) of the peptide conjugates with GraphPad Prism (n=2-3) .

### LogD Determination

The lipophilicity of the radiolabelled triazolopeptides (logD) was determined by the "shake flask method". The radiolabelled conjugates (10 pmol, 10 µL, 1 µM in PBS, ca. 0.25 MBq) were added to a saturated 1:1 mixture of n-octanol/PBS (1 mL, pH 7.4) and shaken vigorously by vortex for 1 min. After centrifugation (3000 rpm, 10 min), 100 µL aliquots of both phases were taken and the radioactivity was measured in a gamma counter (n=2 in quadruplicates).

Biodistribution studies in xenografted mice All procedures were approved by the regional animal committee and were in accordance with international guidelines on the ethical use of animals. Six-week old female CD1 nu/nu mice (Charles River Laboratory, Germany) were inoculated with 5 Mio MZCRC cells. They were grown for 2 weeks until they had reached a size of 50-200 mm2.
On the day of the experiment, they were injected with 10 pmol of the respective ¹⁷⁷Lu-labeled compound in 100 µl PBS (ca. 0.5 MBq) via the tail vein. The mice were sacrificed by CO₂ suffocation 4 hours p.i., and the organs (blood, heart, lungs, spleen, kidneys, pancreas, stomach, intestines, liver, muscle, bone, tumour) were harvested by dissection, weighed and measured in a gamma counter (n=4).
For blocking experiments, the mice were injected with 100 mg of minigastrin (ca. 60'000 pmol, 6000 fold excess) in 100 µl PBS prior to the injection of the radiolabelled compounds (n=4). Tissue distribution data were calculated as percent injected activity per gram of tissue (% ID/g) and statistical analysis was performed with GraphPad Prism.

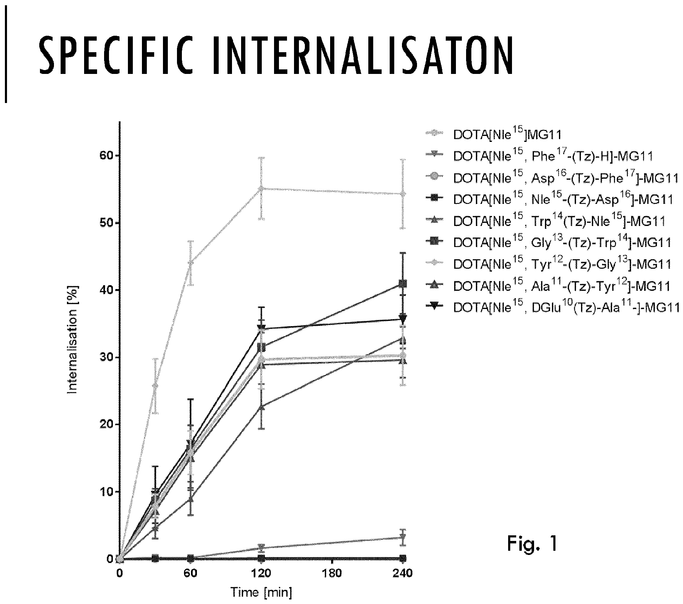

Figure 1 illustrates the specific internalization for the the minigastrin derivate DOTA[Nle15]-MG11 as a reference and the derivated minigastrins having incorporated Tz (1,4-disubstituted 1,2,3-triazole) at different positions in the chain of the last six amino acides. DOTA[Nle15, Tyr¹²-(Tz)-Gly¹³]-MG11 shows a value of more than 50% of specific internalization after 120 minutes.

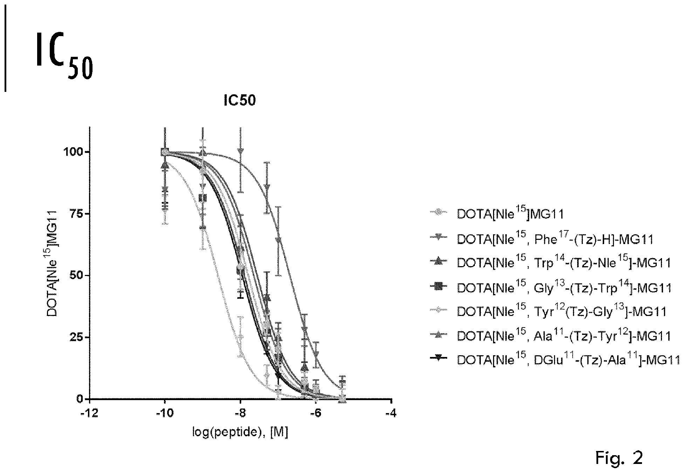

Figure 2 illustrates the half maximal inhibitory concentration IC₅₀ of [Nle¹⁵]-MG11 in its derivated forms according to the termed triazole-scan as compared to the reference minigastrin derivate DOTA[Nle15]-MG11. Accordingly, DOTA[Nle15, Tyr¹²-(Tz)-Gly¹³]-MG11 is the minigastrin derivate having the highest effectiveness in displacing the radiolabelled reference compound PP-F11-N.

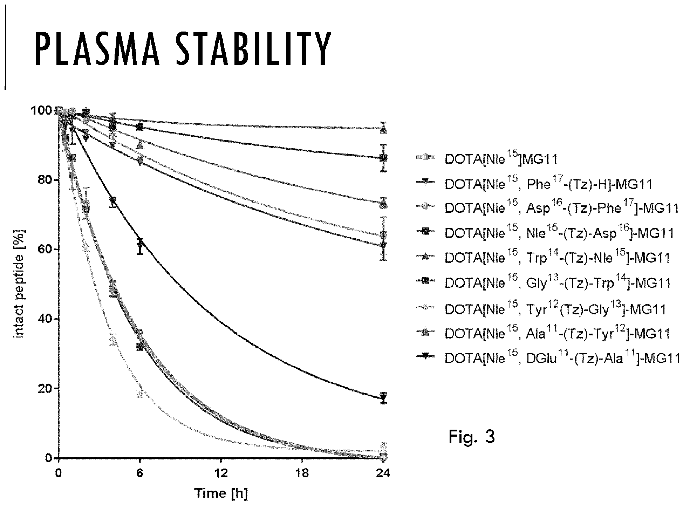

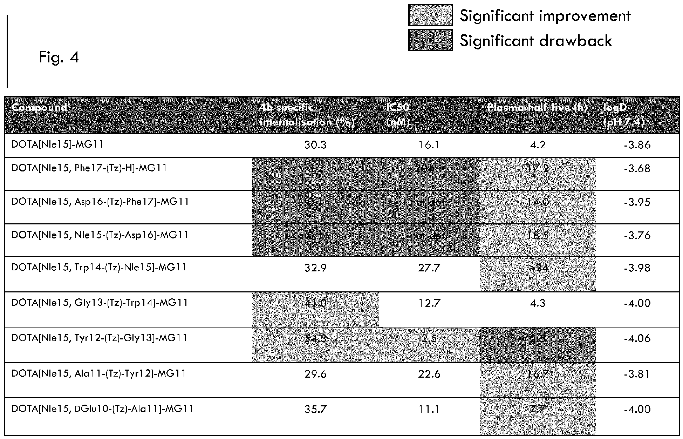

Figure 3 illustrates the plasma stability of [Nle¹⁵]-MG11 in its derivated forms according to the termed triazole-scan as compared to the reference minigastrin derivate DOTA[Nle15]-MG11. Most of the derivates behave similar to the reference derivate. DOTA[Nle15, Tyr¹²-(Tz)-Gly¹³]-MG11 has in this regard a lower stability but nevertheless more than 60% of the peptides remain stable for at least more than 2 hours which allows for a brought range of diagnostic and therapeutic applications. Therefore, as also illustrated in Figure 4 in the summary of the results displayed in Figures 1 to 3, DOTA[Nle15, Tyr¹²-(Tz)-Gly¹³]-MG11 seems to be the best candidate for future diagnostic and therapeutic applications since the highest measured specific internalization and the lowest IC₅₀ value compensate the slightly lower plasma half live.

In comparison to the minigastrin derivate PPF11N from WO 2015/067473 A1, the minigastrin derivate DOTA[Nle15, Tyr¹²-(Tz)-Gly¹³]-MG11 possesses a higher specific internalization at time spans in the range up to 120 min and therefore a higher effectiveness to "block" the addressed receptors (e.g. GPCRs).

## Claims

1. A minigastrin derviate having the formula:
X-Z-Ala-Tyr-Gly-Trp-Met-Asp-Phe-NH₂ (Y),
wherein at least one of the connecting or terminal amide bonds between, before or after the amino acids of the sequence Z, Ala, Tyr, Gly, Trp, Met, Asp, Phe and NH₂ or Y (C-terminal) is replaced by a 1,4-disubstituted or a 1,5-disubstituted 1,2,3-triazole, while X stands for a chemical group attached to the peptide for the purpose of diagnostic and/or therapeutic intervention at CCK-2 receptor relevant diseases, Y stands for C-terminal modifications of the peptide, such as amide, primary and secondary amides, free carboxylic acids and carboxylic ester derivatives including but not limited to amides and esters derived from linear or branched alkyl-,alkenyl-, alkynyl- aromatic-, and heterocyclic alcohols, and Z stands for a linker or DGlu* wherein DGlu* stands for a chain of DGlu having 1 to 6 repetitions (-DGlu- to -DGlu-DGlu-DGlu-DGlu-DGlu-DGlu-).

2. The minigastrin derivate according to claim 1,
wherein methionine is replaced by norleucine, preferably to give minigastrin derivate [Nle¹⁵]-MG11 having one DGlu only and/or to give a minigastrin derivate PP-F11N.

3. The minigastrin derivate according to claim 1 or 2, wherein X stands for a radionuclide including a chelator for radiometals.

4. The minigastrin derivate according to claim 3,
wherein the chelator for radiometals is selected from a group consisting of DOTA (1,4,7,10-tetraazacyclododecane-1,4,7,10-tetraacetic acid), NOTA, NOTAGA, CHX-A"-DTPA and TCMC.

5. The minigastrin derivate according to claim 3, wherein the radionuclide is selected from the group consisting of ¹⁷⁷Lu, ⁹⁰Y, ¹¹¹In, Ga-68/67, Tc-99m, Cu-64/67, Ac-225, Bi-213, Pb-212 and Th-227.

6. The minigastrin derivate according to claim 2, wherein PP-F11N and [Nle¹⁵]-MG11 are defined as:
DOTA-DGlu-DGlu-DGlu-DGlu-DGlu-DGlu-Ala-Tyr-Gly-Trp-Nle-Asp-Phe-NH₂ and
DOTA-DGlu-Ala-Tyr-Gly-Trp-Nle-Asp-Phe-NH₂ resp.

7. The minigastrin derivate according to claim 6, wherein DOTA-DGlu-DGlu-DGlu-DGlu-DGlu-DGlu-Ala-Tyr-Gly-Trp-Nle-Asp-Phe-NH2 is labelled with ¹⁷⁷Lu or DOTA-DGlu-Ala-Tyr-Gly-Trp-Nle-Asp-Phe-NH₂ is labelled with ¹⁷⁷Lu.

8. The minigastrin derivate according to claim 1 or 2, wherein X stands for an optically active chemical compound.

9. The minigastrin derivate according to claim 1 or 2, wherein X stands for a chemotherapeutic active compound or any other therapeutic active compound like tyrosine kinase inhibitor or immunogenic active compound.

10. The minigastrin derivate according to claim 1 or 2, wherein X stands for a nanoparticle or a liposome which have a diagnostic function (e.g. optical active or MRI contrast agent) or which have therapeutic function by themselves or are loaded with an active compound.
